# EUROPÄISCHE PATENTSCHRIFT

(11) **EP 1 910 267 B1**
(45) Veröffentlichungstag und Bekanntmachung des Hinweises auf die Patenterteilung: **11.01.2012**
(21) Anmeldenummer: 06760798.6
(22) Anmeldetag: 24.07.2006
(51) Int. Cl.: C07C 67/03, C07C 69/52, C11C 3/00, C10L 1/18, B01J 23/02

(54) **VERFAHREN ZUR HERSTELLUNG VON CARBONSÄUREALKYLESTERN**
METHOD FOR PRODUCTION OF CARBOXYLATE ALKYL ESTERS
PROCEDE DE PRODUCTION D'ALKYLESTERS D'ACIDE CARBOXYLIQUE

(30) Priorität: 25.07.2005 AT 12452005
(43) Veröffentlichungstag der Anmeldung: 16.04.2008
(73) Patentinhaber: BDI - BioEnergy International AG, 8074 Grambach/Graz (AT); Koncar, Michael, 8501 Lieboch (AT)
(72) Erfinder: GLASL, Wolfgang, A-8010 Graz (AT); MITTELBACH, Martin, A-8010 Graz (AT); SIEBENHOFER, Matthaeus, A-8042 Graz (AT); JEITLER, Erich, A-8230 Hartberg (AT); HAMMER, Wilhelm, A-8074 Grambach/Graz (AT); GÖSSLER, Helmut, A-8042 Graz (AT); KONCAR, Michael, 8501 Lieboch (AT)
(74) Vertreter: Schwarz, Albin
(86) Internationale Anmeldenummer: PCT/AT2006/000311
(87) Internationale Veröffentlichungsnummer: WO 2007/012097

(56) Entgegenhaltungen:
- CH-A- 481 045
- DE-A1- 2 824 782
- DE-A1- 19 942 541
- US-A- 6 124 486
- DATABASE CA [Online] CHEMICAL ABSTRACTS SERVICE, COLUMBUS, OHIO, US; DOMIDE, TEODOR ET AL: "Manufacture of epoxy resin esters of fatty acids or rosin" XP002408436 gefunden im STN Database accession no. 112:99953 & RO 95 945 B1 (CENTRUL DE CERCETARI PENTRU PROTECTII ANTICOROSIVE LACURI SI VOPSELE,) 15. September 1988 (1988-09-15)

## Beschreibung

Die Erfindung betrifft ein Verfahren zur Herstellung von Carbonsäureestern durch Veresterung von Carbonsäuren oder Umesterung von Carbonsäureestern mit Methanol oder Ethanol in Gegenwart eines flüssigen Metallkatalysators.

Unter Umesterung ist die Alkoholyse von Triglyceriden zu verstehen, also die Umsetzung mit niederen Alkoholen, insbesondere Methanol und Ethanol, wobei über die Zwischenprodukte Di- und Monoglyceride die Monoester der Fettsäuren sowie Glycerin entstehen.

Unter dem Begriff "Triglyceride" werden Ester von höheren, gesättigten und/oder ungesättigten Fettsäuren mit Glycerin verstanden. Derartige Ester sind z.B. Hauptbestandteile von Ölen und Fetten pflanzlichen oder tierischen Ursprungs. Viele Fette natürlichen Ursprungs, aber auch gebrauchte Abfallfette und Alt-Speiseöle, enthalten freie Fettsäuren in einem mehr oder minder großen Ausmaß. Diese Fette sind somit ein Gemisch aus Triglyceriden, freien Fettsäuren und anderen Komponenten, wobei der Hauptbestandteil dieses Gemisches in der Regel die Triglyceride sind.

Fettsäureester, insbesondere die Methylester, sind wichtige Zwischenprodukte in der Oleochemie. Allein in Europa werden jährlich 200.000 Tonnen Pflanzenölmethylester als Rohstoffe vor allem für Tenside hergestellt. Daneben gewinnt der Fettsäuremethylester als Kraftstoff für Selbstzündermotoren immer mehr an Bedeutung.

Als Katalysatoren für die Umesterung können basische Katalysatoren (Alkalihydroxide, -alkoholate, -oxide, -carbonate, Anionenaustauscher), saure Katalysatoren (Mineralsäuren, p-Toluolsulfonsäure, Bortrifluorid, Kationenaustauscher) und Enzyme (Lipasen) verwendet werden. Bevorzugt werden heute im Reaktionsgemisch lösliche Katalysatoren verwendet. Diese bilden ein homogenes Gemisch und gewährleisten schnelle Umsatzraten und milde Reaktionsbedingungen. Die am häufigsten verwendeten homogenen Katalysatoren sind Natrium- und Kaliumhydroxid sowie Natriummethylat, welche in Alkohol gelöst dem Pflanzenöl zugemischt werden. Ein derartiges Verfahren ist aus der AT-B 386 222 bekannt. Die saure Katalyse erfordert höhere Reaktionstemperaturen und -drücke und eine aufwendigere Reaktionsführung. Eine saure Umesterung ist aus der FR-A - 85 02340 bekannt.

Die Umesterung mit basischer Katalyse wird im allgemeinen ohne Verwendung eines Lösungsmittels durchgeführt. Die Reaktion beginnt mit einem Zweiphasensystem aus Triglycerid und Alkohol, mit zunehmendem Reaktinsfortschritt und Bildung von Ester entsteht aber eine homogene Phase, welche durch Bildung und Ausscheidung von Glycerin wiederum zweiphasig wird, wobei die leichte Phase der rohe Fettsäurealkylester und die schwere Phase eine glycerinreiche Phase ist.

Aus der EP-A 1 477 551 sind heterogene Katalysatorsysteme bekannt, die aus Salzen von Übergangsmetallen, u.a. Mangan, bestehen. Ferner wird ein Katalysatorsystem aus Natrium/Kaliumhydroxid und Natrium/Kaliumseifen beschrieben.

In der DE-A 19949718 ist eine Umesterungen in Gegenwart von Übergangsmetallseifen beschrieben. Auch die EP-A 1 308 498 beschreibt Ver- und Umesterungsreaktionen in Gegenwart von Alkaliseifen. Als mögliche Katalysatoren werden auch Zinkseifen als genannt.

Aus der US-A 6,818,026 ist ein Verfahren bekannt, bei welchem bei überkritischen Zuständen in der Anwesenheit von festen Katalysatoren gearbeitet wird. Als Katalysator wird u.a. auch Magnesiumoxid genannt. Als Umesterungsraten mit Magnesiumoxid werden jedoch nur 91 % angegeben.

In der US-A 6,147,196 wird ein Umesterungsprozess in drei Stufen beschrieben, wobei zwei Stufen eine heterogen katalysierte Umesterung und eine Stufe eine Destillation der Ester und die Abtrennung eines Sumpfproduktes und Rückführung des Sumpfproduktes in die erste Stufe betreffen. Als Katalysatoren wird Zinkaluminat eingesetzt, der Katalysator gelangt nicht in die Destillation und wird nicht mit dem Sumpfprodukt zurückgeführt.

Die US-A 6,187,939 wiederum beschreibt ein katalysatorloses Verfahren im überkritischen Bereich.

Die DE-A 19942541 beschreibt Veresterungen und Umesterungen von Carbonsäuren und Carbonsäureestern in Gegenwart von Schwermetallverbindungen als Katalysator.

In RO95945 und Database Chemical Abstracts XP002408436, gefunden in STN Database accession no. 112:99953 ist diem Veresterung von Expoxidharzen mit Fettsäuren unter Verwendung von Mg- oder Ca-carbonaten geoffenbart.

US 6,124,486 offenbart die Umesterung von Triglyceriden mit einem Triglycerid unter Verwendung von Ca-stearat als Katalysator.

In DE 28 24 782 ist die Umesterung von Methylacrylat zu Glycerinaldehyd unter Verwendung von Ca-stearat als Katalysator geoffenbart.

CH 481 045 offenbart die Umesterung eines Methylesters zu einem Phenylester.

Die Verfahren des Standes der Technik haben insbesondere den Nachteil, daß die Umsetzung mit Verlusten abläuft, weil u.a. noch freie Fettsäuren im Reaktionsgemisch vorhanden sind. Hier setzt nun die vorliegende Erfindung an, die sich zum Ziel setzt, diesen Nachteil zu beseitigen.

Das erfindungsgemäße Verfahren zur Herstellung von Carbonsäureestern durch Veresterung von Carbonsäuren oder Umesterung von Carbonsäureestern mit Methanol oder Ethanol in Gegenwart eines flüssigen Metallkatalysators ist dadurch gekennzeichnet, daß der flüssige Metallkatalysator das Erdalkalimetallsalz einer Carbonsäure ist und dass der Metallkatalysator nach Beendigung der Ver- bzw. Umesterung für eine weitere Ver- bzw. Umesterung verwendet wird.

Das Erdalkalimetall ist bevorzugt Magnesium und die Carbonsäure ist bevorzugt eine aliphatische Carbonsäure mit insbesondere 10 bis 24 Kohlenstoffatomen im Molekül.

Als Alkohol wird Methanol oder Ethanol eingesetzt.

Als ver- bzw. umzuesternde Carbonsäureester werden insbesondere Triglyceride, welche bevorzugt Fette und Öle pflanzlichen oder tierischen Ursprungs, insbesondere gebrauchte Speiseöle und Abfallfette, sind.

Im erfindungsgemäßen Verfahren werden Carbonsäuren und Carbonsäureester, z.B. Fette und/oder Öle pflanzlichen und/oder tierischen Ursprungs, mit Alkoholen, z.B. aus der Gruppe der einwertigen C 1 bis C4 Alkohole, zu Carbonsäurealkylestern verestert und/oder umgeestert.

Die Katalysatoren können vor der Ver- bzw. Umesterungsreaktion durch Umsetzen von anorganischen Metallverbindungen, z.B. Metalloxiden und/oder Metallhydroxiden, mit Carbonsäuren, z.B. Fettsäuren, gebildet werden.

Die Reaktion findet vorzugsweise bei erhöhter Temperatur statt. Im speziellen bei Temperaturen über 150°C, vorzugsweise über 200°C. Der Druck bei der Reaktion entspricht dem Dampfdruck des Gemisches bei der gegebenen Temperatur und kann gegebenenfalls auch höher als dieser Dampfdruck, z.B. bis zu 20 bar höher, eingestellt werden.

Beim erfindungsgemäßen Verfahren werden Umsätze von über 90% in der Veresterung und in der Umesterung erreicht. Nach dem Reaktionsschritt wird das Reaktionsgemisch aufgetrennt, wobei folgende Produkte gewonnen werden: nicht reagierte Alkohole, Carbonsäuren und Carbonsäureester sowie Wasser aus den Einsatzprodukten, durch die Reaktion entstandene Alkohole, Carbonsäureester und Wasser, und der Katalysator. Beispielhaft werden die Verfahrenschritte anhand der Herstellung von Fettsäureestern aus Ölen und/oder Fetten, welche auch freie Fettsäuren und Wasser enthalten können, durch Ver- bzw Umesterung mit Methanol oder Ethanol beschrieben.

Bei der Reaktion werden die Fette und Öle mit dem eingesetzten Alkohol durch Umesterung in Fettsäureester und Glycerin umgewandelt. Die freien Fettsäuren reagieren mit dem eingesetzten Alkohol zu Fettsäureestern und Wasser. Nach der Reaktion werden zunächst der überschüssige Alkohol und das in der Reaktionsmischung vorhandenen Wasser aus dem Reaktionsgemisch abgetrennt. Vorzugsweise geschieht diese Abtrennung durch Verdampfung des Alkohols und des Wassers. Die Abtrennung könnte aber auch erfolgen mit Hilfe von Membranverfahren oder durch adsorptive und extraktive Verfahren.

Nach der Abtrennung von Wasser und Alkohol werden nicht umgesetzte Mono-, Di- oder Triglyceride, der Katalysator (z.B. die Metallseifen) und schwerflüchtige Verunreinigungen aus dem Gemisch abgetrennt. Da die so abgetrennte Mischung den Katalysator enthält, wird sie in weiterer Folge als Katalysatormischung bezeichnet. Diese Abtrennung kann durch Membranverfahren, durch Kristallisationsverfahren, durch Adsorptionsverfahren oder durch Extraktionsverfahren bewerkstelligt werden. Eine Abtrennung des Katalysators alleine ist auch durch Ionentauscher möglich. Vorzugsweise geschieht die Abtrennung aber durch Destillation. Dabei werden in einem Destillationsapparat vorzugsweise bei Unterdruck (0,1 - 10 mbar absolut) die Fettsäureester-Phase und die Glycerinphase als Kopfprodukt von der Katalysatormischung, die als Sumpfprodukt anfällt, abgetrennt.

Gegebenenfalls kann vor der Abtrennung der Katalysatormischung von der Glycerin- bzw. Fettsäureester-Phase das Gemisch aus der Reaktionsstufe mit Metallverbindungen (z.B. Oxiden oder Hydroxiden), welche in der Folge die Metallseifen bilden, versetzt werden. Dabei werden freie Fettsäuren, die im Reaktionsgemisch vorhanden sind, verseift, wodurch die Abtrennung der Katalysatormischung von der Ester- und Glycerinphase erleichtert wird. Diese Zugabe der Metallerbindungen kann auch vor oder nach der Abtrennung von Alkohol und/oder Wasser erfolgen. Über diesen Weg besteht auch eine Möglichkeit, Katalysator in den Prozess einzubringen.

Gegebenenfalls kann vor der Abtrennung der Katalysatormischung eine Trennung zwischen Fettsäureesterphase und Glycerinphase erfolgen und die Abtrennung der Katalysatormischung aus der Fettsäureester-Phase und der Glycerinphase getrennt durchgeführt werden. In diesem Fall können die gleichen Methoden wie oben genannt vorzugsweise die Destillation angewendet werden.

Die Fettsäureester-Phase aus dem Abtrennschritt der Katalysatormischung kann in weiterer Folge Reinigungsprozessen zugeführt werden. Als Reinigungsverfahren eignen sich die Wäsche mit polaren Flüssigkeiten sowie Ionentauscherverfahren, Absorptionsverfahren, Extraktionsverfahren oder weitere Destillationsschritte.

Die gereinigte Fettsäure-Ester-Phase kann z.B. als Kraftstoff verwendet werden. Die nach der Abtrennung der Katalysatormischung erhaltene Glycerinphase kann weiteren Reinigungsschritten zugeführt werden. Diese Reinigungsschritte können Ionentauscherprozesse, absorptive Prozesse, extraktive Prozesse und Destillationsprozesse umfassen. Nach einer Destillation und anschließender Aktivkohlebehandlung kann beispielsweise Pharmaglycerin gewonnen werden.

Die gewonnene Katalysatormischung kann ohne weitere Aufbereitung wieder in eine neue Reaktionsstufe (Ver- bzw. Umesterung) zurückgeführt werden und dort erneut als Katalysator für die Ver- und Umesterungsreaktion wirken. Es kann aber auch der Katalysator aus der Katalysatormischung abgetrennt und in reiner Form rückgeführt werden. Die Abtrennung des Katalysators kann durch Einsatz von Fällungs-, Kristallisations-, Membran-, Ionentauscher-, Adsorptions-, Extraktions-, oder Destillationsverfahren durchgeführt werden. Eine spezielle Möglichkeit ist die Fällung der Alkali-/Erdalkalimetallverbindungen mit Wasser. Durch Oxidation der Katalysatormischung können die organischen Bestandteile dieser Mischung zerstört werden und die Metalle als anorganische Verbindungen in oder vor die Reaktionsstufe zurückgeführt werden. Diese Metallverbindungen werden durch Carbonsäuren wieder in die benötigten Katalysatorverbindungen umgewandelt.

Die gewonnene Katalysatormischung kann entweder zur Gänze oder teilweise zurückgeführt werden. Ein teilweises Ausbringen der Katalysatormischung hat den Vorteil, dass damit auch Verunreinigungen aus dem Prozess ausgebracht werden.

Die Rückführung kann entweder direkt in den Reaktionsraum oder in die Einsatzprodukte vor deren Zuführung in den Reaktionsraum erfolgen.

Der ersten Reaktionsstufe zur Ver- und Umesterung können weitere Reaktionsstufen folgen, wobei vorzugsweise die gebildeten Nebenprodukte (hauptsächlich Wasser und gebildete Alkohole) nach jeder Reaktionsstufe abgetrennt werden können und gegebenenfalls in jeder Reaktionsstufe neuer Katalysator sowie neuer Alkohol, der Reaktionsmischung zugesetzt wird. Die Reaktion kann diskontinuierlich oder kontinuierlich geführt sein, wobei aus energetischer Sicht die kontinuierliche Prozessführung mit Wärmerückgewinnung zu bevorzugen ist.

Das erfindungsgemäße Verfahren bietet vor allem bei der Herstellung von Fettsäureestern aus pflanzlichen und/oder tierischen Fetten und/oder Ölen durch Veresterung und oder Umesterung mit Alkoholen, z.B. aus der Gruppe der C1 bis C4 Alkohole große Vorteile. Gegenüber dem in der DE-A 19942541 beschriebenen Verfahren weist das erfindungsgemäße Verfahren deutlich bessere Umsatzraten auf. Die Vergleichsbeispiele zeigen auch, dass vor allem mit Magnesiumseifen die Veresterung sehr gut abläuft und fast keine freien Fettsäuren im Reaktionsgemisch vorhanden sind. Dies bringt Vorteile in der Auftrennung des Reaktionsgemisches, weil z.B. bei der Destillation freie Fettsäuren mit den Fettsäureestern teilweise in das Kopfprodukt ausgetragen werden, wodurch die Qualität der Fettsäureester verschlechtert wird.

Die meisten gängigen Verfahren zur Herstellung von Fettsäureestern aus Fetten und Ölen durch Umesterung mit Alkohol arbeiten mit basischen Katalysatoren. Diese Verfahren können nur mit weitgehend wasserfreien und fettsäurefreien Rohstoffen arbeiten. Bei diesen Prozessen wird der Katalysator zerstört, es muss immer wieder neuer Katalysator zugeführt werden, die Kosten dafür sind entsprechen hoch. Für KOH z.B. muss man mit Katalysatorkosten in der Höhe von ca. 7 bis 9 Euro pro Tonne Fettsäureester rechnen. Beim erfindungsgemäßen Verfahren aber wird der Katalysator zurückgewonnen, wodurch die Katalysatorkosten beim gegenständlichen Verfahren unter 1 Euro pro Tonne Fettsäureester liegen.

Das gegenständliche Verfahren hat den Vorteil, dass im Ausgangsgemisch auch freie Fettsäuren in beliebigen Konzentrationen vorliegen können. Diese freien Fettsäuren werden bei der genannten Reaktion ebenfalls zu Fettsäurealkylestern verestert. Dadurch sind auch Fette/Öle minderer Qualität verarbeitbar. Ein weiterer entscheidender Vorteil des Verfahrens liegt darin, dass die Ver/Umesterungsreaktion auch in Anwesenheit von Wasser durchgeführt werden kann. Dadurch wird es möglich, auch wasserhältige Rohstoffe, speziell wasserhältige Alkohole einzusetzen.

Nicht umgesetzte freie Fettsäuren oder Glyceride werden ebenfalls wieder der Ver/Umsterung zugeführt, dadurch entstehen keine Verluste.

Gegenüber katalysatorfreien Prozessen bietet das erfindungsgemäße Verfahren den Vorteil, dass die Reaktion bei geringeren überstöchiometrischen Alkoholmengen stattfindet, was wiederum die Wirtschaftlichkeit des Verfahrens deutlich verbessert, weil der Aufwand für die Rückgewinnung des Alkohols geringer ist, als bei Verfahren ohne Katalysator.

Mit dem nachfolgenden Beispiel werden bevorzugte Varianten des erfindungsgemäßen Verfahrens noch näher beschrieben.

### Beispiel 1

In einer Vergleichsreihe wurden unterschiedliche Katalysatoren für die Ver- bzw. Umesterung von Carbonsäureestern getestet. Folgende Chemikalien kamen zum Einsatz:
Rapsöl aus 00 Rapssaat, eritschleimt, entsäuert, desodoriert, getrocknet,
Ölsäure technisch rein,
Methanol technisch rein,
Wasser entionisiert,
Als Katalysatoren in technisch reiner Qualität:
Tetrabutyltitanat,
Dibutylzinndilaurat,
Magnesiumstearat,
Calziumstearat,
Natriumstearat

Mit allen genannten Katalysatoren wurden folgende Versuche durchgeführt:
Jeweils 150 g Rapsöl, 37,5 g Ölsäure und 123 g Methanol wurden mit der in Tabelle 1 angegebenen Katalysatormenge vermischt und in einem Laborautoklaven bei 210 °C über einen Zeitraum von 30 Minuten zur Reaktion gebracht. Nach der Reaktion konnten in der Esterphase die angegebenen Konzentrationen an Fettsäuremethylethylester (FS-Methylester) gemessen werden.

**Tabelle 1**

| Katalysator | Katalysator | Metall-Menge | Triglyceride | Diglyceride | Monoglyceride | freie Fettsäuren | FS-Melhylester |
|---|---|---|---|---|---|---|---|
| | [mol Kat /kg Öl] | [g Met./ kg Öl] | [gew.%] | [gew.%] | [gew.%] | [gew.%] | [gew.%] |
| | | | | | | | |
| Tetrabutyltitanat | 0,066 | 3,2 | 5,5 | 9,7 | 11,3 | 4,8 | 69,0 |
| Magnesiumstearat | 0,134 | 3,3 | 0.3 | 1,8 | 4,3 | 0,1 | 92,2 |
| Dibutylzinndilaurat | 0,027 | 3,2 | 1,1 | 8,8 | 11,8 | 4,2 | 72,7 |
| Calziumstearat | 0,080 | 3,2 | 0,4 | 2,3 | 6,4 | 4,0 | 86,8 |
| Natriumstearat | 0,138 | 3,2 | 9,9 | 14,2 | 13,1 | 6,2 | 56,6 |
| Tetrabutyltitanat | 0,134 | 6,4 | 1,7 | 5,7 | 9,7 | 3,8 | 79,1 |
| Dibutylzinndilaurat | 0,134 | 15,9 | 0,4 | 6,1 | 8,4 | 3,8 | 80,0 |

Es ist ersichtlich, dass bei gleicher Metallmenge im Gemisch mit den Erdalkaliverbindungen deutlich bessere Umsätze erzielbar sind. Dies gilt sowohl für Gewichtsverhältnisse, als auch für Molverhältnisse.

Das gesamte Reaktionsprodukt wurde in eine Labordestille gefüllt. In dieser Destille wurden zunächst bei Normaldruck Methanol und Wasser aus dem Reaktionsgemisch abdestilliert. Dann wurde bei einem Druck von ca. 0,5 mbar die Hauptmenge der Methylesterphase und der Glycerinphase destillativ abgetrennt. Die metallorganischen Verbindungen, die als Katalysator wirken, verblieben bei dieser Destillation im Sumpfrückstand. Dieser Rückstand wurde nun erneut mit Rapsöl und Ölsäure sowie mit Methanol vermischt und erneut einer Ver/Umesterungsreaktion zugeführt. Die gewählten Mengenverhältnisse können der Tabelle 2 entnommen werden.

**Tabelle 2**

| Katalysator | Öl, Fettsäuren, Katalysatormischung | Methanol | Zusammensetzung Esterphase nach der Reaktion | | | | |
|---|---|---|---|---|---|---|---|
| | | | Triglyceride | Diglyceride | Monoglyceride | freie Fettsäuren | Fettsäureester |
| | [g] | [g] | [gew.%] | [gew.%] | [gew.%] | [gew.%] | [gew.%] |
| | | | | | | | |
| Tetrabutyltitanat | 190,79 | 124,0 | 1,6 | 5,9 | 9,5 | 4,7 | 78,2 |
| Magnesiumstearat | 187,55 | 121,9 | 0,1 | 2,5 | 5,0 | 0,1 | 92,4 |
| Dibutylzinndilaurat | 193,3 | 125,7 | 2,5 | 6,5 | 9,8 | 4,8 | 76,4 |
| Calziumstearat | 187,74 | 122,1 | 0,1 | 2,3 | 6,4 | 4,0 | 86,8 |
| Natriumstearat | 182,76 | 118,8 | 1,8 | 7,2 | 14,2 | 4,0 | 72,8 |

Die Bedingungen für die zweite Reaktionsstufe wurde wiederum so gewählt, dass das Produkt für einen Zeitraum von 30 Minuten auf einer Temperatur von 210 °C gehalten wurde. Der Methylestergehalt in der Methylesterphase nach dieser zweiten Reaktionsstufe ist ebenfalls in Tabelle 2 dargestellt. Der höhere Umsatz mit den Erdalkalimetallkatalysatoren ist deutlich erkennbar.

Auch nach der zweiten Reaktion wurde das Gemisch destillativ getrennt (Abtrennung von Methanol und Wasser), und danach erfolgte die Destillation von Methylester und Glycerin. Die Esterphasen aus beiden Destillationen wurden jeweils von den Glycerinphasen getrennt (durch Schwerkraftabsetzung) und in der Folge mit 0,5 Gew% Wasser gewaschen. Der so erhaltene Ester entsprach den Qualitätsanforderungen der EN 14214. Die Ausbeute, die mit den einzelnen Katalysatoren erreicht werden konnte, ist in Tabelle 3 zusammengefasst. Es ist deutlich zu sehen, dass mit den Erdalkalikatalysatoren eine viel bessere Ausbeute erzielt wurde als mit den Schwermetallkatalysatoren.

**Tabelle 3**

| Ausbeute Fettsäuremethylester bezogen auf die eingesetzte Öl- und Fettsäuremenge | |
|---|---|
| Tetrabutyltitanat | 74% |
| Magnesiumstearat | 94% |
| Dibutylzinndilaurat | 77% |
| Calziumstearat | 88% |
| Natriumstearat | 68% |

### Beispiel 2

In 2 Versuchsreihen in einer kontinuierlich arbeitenden Technikumsanlage wurden über einen Zeitraum von jeweils 5 Tagen folgende Rohstoffe verarbeitet: Altspeiseöl mit einem Gehalt an freien Fettsäuren von ca. 7% (Altspeiseöl: gebrauchte Speiseöle aus Haushalten und Gewerbebetrieben enthalten eine Mischung an unterschiedlichen pflanzlichen und tierischen Fetten und Ölen, freie Fettsäuren, Wasser, sonstige Verunreinigungen, z.B. aus Frittierprozessen), und Tierfett aus einer Tierkörperverwertungsanlage mit einem Gehalt an freien Fettsäuren von ca. 14% verarbeitet.

Als Katalysator wurde Magnesiumoleat eingesetzt. Zur Herstellung des Katalysators wurden 4 kg Magnesiumoxid und 44 kg Ölsäure bei ca. 60°C für einen Zeitraum von 2 Stunden miteinander zur Reaktion gebracht und danach für die Verarbeitung der Öle bzw Fette bereit gehalten.

Die Verarbeitung umfaßte folgenden Schritte: Zuerst wurde das Gemisch Öl/Fett mit Methanol und dem Katalysator vermischt. Dieses Gemisch wurde aus dem Anmischbehälter mit Hilfe einer Pumpe in einen Vorlagebehälter gepumpt. Von diesem Vorlagebehälter wurde das Gemisch mit Hilfe einer weiteren Pumpe kontinuierlich mit einem Durchsatz von 251/h in die Anlage gepumpt. Zunächst wurde das Gemisch durch einen Wärmetauscher geleitet und auf 215 °C erhitzt. Nach dem Wärmetauscher strömte das Gemisch weiter durch einen Reaktor, die Verweilzeit im Reaktor betrug 30 Minuten, der Druck wurde mit Hilfe eines nach dem Reaktor angeordneten Drosselventils auf 50 bar abs eingestellt.

Im Wärmetauscher und im Reaktionsbehälter liefen die gewünschten Veresterungsrealctionen (freie Fettsäuren und Methanol zu Fettsäuremethylester und Wasser) und Umesterungsreaktionen (Glyceride und Methanol zu Fettsäuremethylester und Glycerin) ab. Über das Drosselventil wurde das Reaktionsgemisch auf atmosphärischen Druck entspannt, wobei Methanol und Wasser verdampften und im anschließenden Flashbehälter abgetrennt wurden. Das verbleibende Gemisch wurde in einer weitere Entgasungsstufe geleitet, die bei 140°C und 50 mbar abs betrieben wurde, wodurch restliche Mengen an Wasser und Methanol abgetrennt wurden.

Nach dieser Entgasung wurde das verbleibende Gemisch kontinuierlich in einer Kurzwegdestillationsanlage aufgetrennt in ein Sumpfprodukt, welches hauptsächlich nicht reagierte Glyceride, Fettsäuren, den Katalysator und geringe Mengen an Methylester enthielt, und in ein Kopfprodukt, welches in Form von zwei flüssigen Phasen, einer Methylesterphase und einer Glycerinphase, anfiel. Diese Phasen wurden durch einen Schwerkraftabscheider geleitet und so voneinander getrennt. Die Glycerinphase enthielt ca. 99,5% Glycerin. Die Methylesterphase wurde in einem Mischbehälter mit 0,5 Gew% Wasser gewaschen, die Wasserphase wurde in einem Schwerkraftabscheider abgetrennt und die Esterphase anschließend in einem Flashbehälter bei 120°C und ca. 100 mbar abs getrocknet. Danach lag der Methylester in einer Qualität vor, die der Norm EN 14214 entsprach. Lediglich der geforderte CFPP Wert konnte beim Tierfettester und beim Altspeisefett für den Winter nicht erreicht werden. Das Sumpfprodukt wurde in einem Behälter gesammelt und in der Folge im Anmischbehälter wieder mit weiterem Rohstoff vermischt und so neuerlich für die Katalyse der genannten Reaktionen verwendet.

Dieser Prozess wurde für jeden Rohstoff über einen Zeitraum von 5 Tagen über 24 Stunden kontinuierlich betrieben. Die in der nachfolgenden Tabelle 4 zusammengefaßten Mengen wurden verarbeitet bzw. gewonnen:

**Tabelle 4**

| | Einsatz | | | | Produkte | | | | | Methylester |
|---|---|---|---|---|---|---|---|---|---|---|
| | Öl/Fett | Methanol | Katalysator | Wasser | Methylester | Glycerin | Abwasser | Methanol | Sumpf | Ausbeute |
| Altspeiseöl | 846,0 | 691,7 | 10,2 | 7,6 | 820,0 | 80,0 | 8,3 | 600,4 | 47,0 | 96,9% |
| Tierfett | 842,0 | 615,4 | 10,4 | 7,2 | 827,0 | 81,5 | 7,9 | 534,3 | 31,0 | 98,2% |

Das Altspeiseöl wies einen Polymergehalt von 5,5% auf. Da die Ausbeute an Fettsäuremethylester bei Altspeiseöl fast 97% beträgt, wurden diese Polymere großteils auch zu Methylester umgesetzt. Im Sumpfprodukt lag die Polymerkonzentration unter 1%.

### Vergleichsbeispiel

Die im Beispiel 2 angegebenen Rohstoffmengen Altspeiseöl und Tierfett wurden nach dem Stand der Technik mit Kaliummethanolat und Methanol vermischt, um eine Umesterung zum jeweiligen Methylester durchzuführen. Dazu wurde zunächst Kaliumhydroxid (techn., ca. 88%) in Methanol (techn. rein) aufgelöst. Die Mengen wurden so gewählt, dass eine Lösung mit einer KOH-Konzentration von 8% erhalten wurde. Diese Lösung wurde mit den genannten Fetten im Verhältnis Öl: Lösung = 10 : 1,5 vermischt. Nach einer Reaktionszeit von 20 Minuten bei ca. 40°C und einer anschließenden Absetzzeit von 12 Stunden fand keine Phasentrennung statt, der Katalysator Kaliummethylat war durch Verseifungsreaktionen mit den in den Rohstoffen vorhandenen Fettsäuren inaktiv geworden. Nach Zugabe von konzentrierter Schwefelsäure bis zu einem pH-Wert von 4 im Reaktionsgemisch trennte sich das jeweilige Reaktionsgemisch in eine Öl/Fettphase und eine Methanol/Glycerinphase auf. Die Ausbeute an Methylester in der Öl/Fettphase lag nur bei ca. 15%.

## Patentansprüche

1. Verfahren zur Herstellung von Carbonsäureestern durch Veresterung von Carbonsäuren und/oder Umesterung von Carbonsäureestern mit Methanol oder Ethanol in Gegenwart eines flüssigen Metallkatalysators,
**dadurch gekennzeichnet,**
**daß** der flüssige Metallkatalysator das Erdalkalimetallsalz einer Carbonsäure ist und daß der Metallkatalysator nach Beendigung der Ver- bzw. Umesterung für eine weitere Ver- bzw. Umesterung verwendet wird.

2. Verfahren nach Anspruch 1, dadurch gekenzeichnet, daß das Erdalkalimetall Magnesium ist.

3. Verfahren nach einem der Anspruche 1 oder 2, **dadurch gekennzeichnet, daß** die Carbonsäure eine aliphatische Carbonsäure ist.

4. Verfahren nach Anspruch 3, **dadurch gekennzeichnet, daß** die aliphatische Carbonsäure 10 bis 24 Kohlenstoffatome besitzt.

5. Verfahren nach einem der Ansprüche 1 bis 4, **dadurch gekennzeichnet, daß** als ver- bzw. umzuesternde Carbonsäureester Triglyceride eingesetzt werden.

6. Verfahren nach Anspruch 5, **dadurch gekennzeichnet, daß** als Triglyceride, Fette und Öle pflanzlichen oder tierischen Ursprungs, insbesondere gebrauchte Speiseöle und Abfallfette, eingesetzt werden.

## Claims

1. A process for the production of carboxylic acid esters by esterification of carboxylic acids and/or transesterification of carboxylic acid esters with methanol or ethanol in the presence of a liquid metal catalyst,
**characterized in that**
the liquid metal catalyst is the alkaline earth metal salt of a carboxylic acid and that, upon completion of the esterification or transesterification, respectively, the metal catalyst is used for another esterification or transesterification, respectively.

2. The process according to claim 1, **characterized in that** the alkaline earth metal is magnesium.

3. The process according to any of claims 1 or 2, **characterized in that** the carboxylic acid is an aliphatic carboxylic acid.

4. The process according to claim 3, **characterized in that** the aliphatic carboxylic acid comprises 10 to 24 carbon atoms.

5. The process according to any of claims 1 to 4, **characterized in that** triglycerides are used as the carboxylic acid esters to be esterified or transesterified, respectively.

6. The process according to claim 5, **characterized in that** fats and oils of a vegetable or animal origin, especially used edible oils and waste fats, are used as triglycerides.

## Revendications

1. Procédé de production d'esters d'acides carboxyliques par estérification d'acides carboxyliques et/ou par trans-estérification d'esters d'acides carboxyliques avec du méthanol ou de l'éthanol en présence d'un catalyseur métallique liquide
**caractérisé en ce**
**que** le catalyseur métallique liquide est un sel de métal alcalino-terreux d'un acide carboxylique et que le catalyseur métallique est employé après l'achèvement de l'estérification, respectivement de la trans-estérification, pour une nouvelle estérification, respectivement trans-estérification.

2. Procédé selon la revendication 1 **caractérisé en ce que** le métal alcalino-terreux est du magnésium.

3. Procédé selon l'une des revendications 1 ou 2 **caractérisé en ce que** l'acide carboxylique est un acide carboxylique aliphatique.

4. Procédé selon la revendication 3 **caractérisé en ce que** l'acide aliphatique possède de 10 à 24 atomes de carbone.

5. Procédé selon l'une des revendications de 1 à 4 **caractérisé en ce qu'**en tant esters d'acides carboxyliques à estérifier, respectivement à trans-estérifier, des triglycérides sont mis en oeuvre.

6. Procédé selon la revendication 5 **caractérisé en ce qu'**en tant que triglycérides, des graisses et des huiles d'origine végétale ou animale, en particulier, des huiles alimentaires déjà utilisées et des graisses de récupération soient mises en oeuvre.
